(19) Europäisches Patentamt
European Patent Office
Office européen des brevets

(11) **EP 4 223 205 A1**

(12) **EUROPÄISCHE PATENTANMELDUNG**

(43) Veröffentlichungstag:
**09.08.2023   Patentblatt 2023/32**

(21) Anmeldenummer: **23155157.3**

(22) Anmeldetag: **06.02.2023**

(51) Internationale Patentklassifikation (IPC):
**A61B 1/00** *(2006.01)*      **A61B 1/313** *(2006.01)*

(52) Gemeinsame Patentklassifikation (CPC):
**A61B 1/3132; A61B 1/00096**

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**KH MA MD TN**

(30) Priorität: **07.02.2022   DE 102022102804**

(71) Anmelder: **avateramedical GmbH**
**07745 Jena (DE)**

(72) Erfinder:
• **MARKWEG, Eric**
 **98693 Ilmenau (DE)**
• **HUNZE, Stephan**
 **07645 Stadtroda (DE)**
• **HÜBNER, Matthias**
 **98693 Ilmenau (DE)**

(74) Vertreter: **Gleim Petri Patent- und Rechtsanwaltspartnerschaft mbB Neugasse 13 07743 Jena (DE)**

(54)   **ENDOSKOP FÜR DIE LAPAROSKOPISCHE CHIRURGIE**

(57)   Die Erfindung betrifft ein Endoskop für die laparoskopische Chirurgie, welches einen Endoskopschaft (10) mit einer Mittelachse (8), einem proximalen und einem distalen Ende, und einen ersten optischen Strahlweg mit einem am distalen Ende des Endoskopschafts (10) angeordneten Prisma mit einer Lichteintrittsfläche und einer Lichtaustrittsfläche, einer vom distalen Ende des Endoskopschafts (10) gesehen hinter dem Prisma angeordneten Linse (4) mit einer optischen Achse (11) und einer vom distalen Ende des Endoskopschafts (10) hinter der Linse (4) angeordnete Strahlumlenkungsoptik (5) umfasst. Die Lichteintrittsfläche des Prismas ist dabei senkrecht zur Mittelachse (8) ausgerichtet, und die Lichtaustrittsfläche des Prismas schließt mit einer ersten, orthogonal zur Mittelachse (8) ausgerichteten ersten Raumachse einen vorgegebenen ersten Neigungswinkel ein. Zur Vergrößerung der numerischen Apertur der Linse (4) wird die optische Achse (11) der Linse (4) senkrecht zur Lichtaustrittsfläche des Prismas angeordnet. Im Ergebnis weist die Linse (4) eine Querschnittsfläche auf, die größer als die Lichteintrittsfläche des Prismas ist.

Fig. 2

**Beschreibung**

**Gebiet der Erfindung**

Die Erfindung betrifft ein Endoskop für die laparoskopische Chirurgie, welches einen Endoskopschaft umfasst. Der Endoskopschaft weist eine Mittelachse, ein proximales und ein distales Ende sowie einen ersten optischen Strahlweg mit einem am distalen Ende des Endoskopschafts angeordneten Prisma mit einer Lichteintrittsfläche und einer Lichtaustrittsfläche auf. Vom distalen Ende des Endoskopschafts gesehen hinter dem Prisma ist eine Linse mit einer optischen Achse und vom distalen Ende des Endoskopschafts hinter der Linse eine Strahlumlenkungsoptik angeordnet, wobei die Lichteintrittsfläche des Prismas senkrecht zur Mittelachse ausgerichtet ist, und die Lichtaustrittsfläche des Prismas mit einer ersten, orthogonal zur Mittelachse ausgerichteten ersten Raumachse einen vorgegebenen ersten Neigungswinkel einschließt.

**Stand der Technik**

Minimalinvasive chirurgische Eingriffe werden für verschiedenste Operationen eingesetzt, um Traumata und Narben für den Patienten zu reduzieren. Dabei werden über kleine Inzisionen Zugänge für spezielle Instrumente und ein Endoskop geschaffen, an die Trokare angelegt werden. Diese werden als Führung in die Körperhöhle, in der die Operation stattfindet, verwendet, und dichten die Inzisionen ab. Nach dem Setzen der Trokare wird die Körperhöhle mit einem Gas, in der Regel $CO_2$, gefüllt, um den Raum für die Instrumente zu schaffen.

Endoskope werden dazu verwendet, dem Chirurgen Einblick in das Operationsgebiet zu geben. Moderne Endoskope arbeiten digital und bestehen aus einem Objektiv, einer Strahlumlenkungsoptik und einem Sensor, wobei zwischen starren und flexiblen Endoskopen unterschieden wird.

Starre Endoskope weisen einen Metallschaft auf und können Optiken umfassen, die sich über die gesamte Länge des Schafts erstrecken. Die Bildaufnahme erfolgt dann über einen oder mehrere Photodetektoren am proximalen Ende des Schafts. Alternativ werden die Photodetektoren nah am distalen Ende platziert, so dass die optische Strecke kurzgehalten werden kann.

Das Auflösungsvermögen eines Endoskops wird maßgeblich durch seine numerische Apertur bestimmt, welche vom Brechungsindex des zwischen Objektiv und Probe befindlichen Materials und dem Öffnungswinkel abhängt. Der Öffnungswinkel kann wiederum leicht über den Durchmesser der Linse beeinflusst werden. Dabei kann eine Anordnung von Linsen, die in einem Winkel zum Schaft ausgerichtet sind, vorteilhaft für das Auflösungsvermögen sein. In der Praxis besteht die Notwendigkeit, verschiedene Blickwinkel bereitzustellen, so dass Endoskope mit geraden als auch zum Schaft abgewinkelten Blickrichtungen, z.B. um 30°, eingesetzt werden.

Ein Beispiel für ein solches Endoskop ist in der US 6,817,975 B1 offenbart, wo sowohl ein gerades Endoskop als auch ein abgewinkeltes vorgestellt wird, wobei ein Zwischenbild in einem optischen Element wie z. B. einer Linse abgebildet wird. Auf diese Weise wird das Zwischenbild gewölbt in dem optischen Element abgebildet, so dass im Ergebnis eine vergrößerte numerische Apertur gegenüber einer Optik, in der sich die Zwischenbilder zwischen den optischen Elementen befinden, erreicht wird.

Eine alternative Möglichkeit, das Auflösungsvermögen zu verbessern besteht darin, die Eingangslinse des Endoskops so groß wie möglich zu gestalten. Ein solches System ist in der DE 10 2012 110 905 A1 offenbart. Über ein Deckglas und eine Negativlinse wird hier das einfallende Licht in ein Prisma geleitet, zweimal totalreflektiert und in eine Optik mit kleinerem Durchmesser eingekoppelt. Dadurch können eine höhere numerische Apertur erreicht und hochauflösende Sensoren verbaut werden. Ein solcher Aufbau ist allerdings lediglich für abgewinkelte Endoskope sinnvoll.

**Beschreibung der Erfindung**

Es ist Aufgabe der vorliegenden Erfindung, eine Optik für ein gerades Endoskop bereitzustellen, die eine höhere numerische Apertur und somit ein verbessertes Auflösungsvermögen aufweist.

Diese Aufgabe wird bei dem eingangs beschriebenen Endoskop dadurch gelöst, dass die optische Achse der Linse senkrecht zur Lichtaustrittsfläche des Prismas angeordnet ist und die Linse eine Querschnittsfläche aufweist, die größer als die Lichteintrittsfläche des Prismas ist. Auf diese Weise wird ermöglicht, eine größere Linse mit einer vergrößerten numerischen Apertur zu verwenden, da der Neigungswinkel das Anbringen einer solchen Linse innerhalb des räumlich begrenzten Endoskopschafts erlaubt.

In einer vorteilhaften Ausgestaltung beträgt der erste Neigungswinkel 45°. Auf diese Weise kann die Ausdehnung der Linse in dieser Raumrichtung so angepasst werden, dass ein Kompromiss zwischen Maximierung der Querschnittsfläche und Vermeidung von Abbildungsfehlern erreicht wird. Damit ist es möglich, allgemein den Durchmesser der Linse zu vergrößern. Entsprechend den räumlichen Gegebenheiten innerhalb des Endoskopschafts und der konkreten Auslegung können auch Linsensysteme oder Kitgruppen zum Einsatz kommen. Auch diese sollen unter dem Sammelbegriff einer Linse allgemein erfasst sein.

Um eine Abbildung in bestmöglicher Qualität zu erlauben, ist das Prisma vorteilhafterweise als Bauernfeind-Prisma oder als Schmidt-Prisma ausgebildet, da diese die Abbildung nur geringfügig beeinflussen. Aus demselben Grund ist es von Vorteil, die Strahlumlenkungsoptik als einen Parabolspiegel mit Off-Axis-Strahlführung und/oder als ein weiteres Prisma auszubilden, die den Strahlweg parallel zur Mittelachse ausrichten. So kann das Licht mit einem oder mehreren regulär angeordneten Bildsensoren aufgenommen werden, die, wie fachüblich, senkrecht zum Endoskopschaft angeordnet sind. So wird der Aufbau erleichtert und es werden Verzerrungseffekte in der Abbildung vermieden. Es ist ebenso möglich, die Bildsensoren parallel zu der Mittelachse auszurichten, und mittels der Strahlumlenkungsoptiken das Licht auf diese abzulenken. Der optische Aufbau kann so noch kompakter gestaltet werden.

Um die Querschnittsfläche der Linse weiter zu vergrößern, ist die Lichtaustrittsfläche des Prismas vorzugsweise so ausgerichtet, dass sie mit einer zweiten, orthogonal zur Mittelachse und zur ersten Raumachse ausgerichteten Raumrichtung einen vorgegebenen zweiten Neigungswinkel einschließt, der vorzugsweise 45° beträgt. Dies erlaubt, den für die Größe der Querschnittsfläche der Linse besten Kompromiss zwischen maximaler numerischer Apertur und Abbildungsqualität zu erreichen.

Das Prisma besteht vorzugsweise aus zwei oder mehr Teilprismen, welche hintereinander angeordnet, bevorzugt miteinander verkittet sind. Auf diese Weise können Standardbauteile wie Bauernfeind-Prismen und/oder als Schmidt-Prismen für die Erfindung verwendet werden.

In einer weiteren vorteilhaften Ausgestaltung umfasst das Endoskop einen zweiten optischen Strahlweg, der dem Aufbau des ersten optischen Strahlwegs entspricht, wobei die Anordnung des ersten und des zweiten optischen Strahlwegs symmetrisch zur Mittelachse erfolgt. Auf diese Weise kann ein Stereoendoskop basierend auf dem zugrundeliegenden Konzept der Erfindung realisiert werden. Ein solches ermöglicht eine verbesserte Darstellung des Operationsbereichs.

Um den Bauraum innerhalb des Schafts des Stereoendoskops darauf aufbauend weiter zu optimieren, schneiden sich in einer besonders bevorzugten Ausführung der erste und der zweite optische Strahlweg oder sie werden aneinander vorbeigeführt. Dies ermöglicht, zwischen den optischen Bauteilen mehr Raum vorzusehen, so dass erleichtert wird, die einzelnen Bauteile auszulegen und die Bildqualität betreffende Kompromisse vermieden werden.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

## Kurze Beschreibung der Zeichungen

Nachfolgend wird die Erfindung anhand von Ausführungsbeispielen unter Bezugnahme auf die beigefügten Zeichnungen, die ebenfalls erfindungswesentliche Merkmale offenbaren, noch näher erläutert. Diese Ausführungsbeispiele dienen lediglich der Veranschaulichung und sind nicht als einschränkend auszulegen. Beispielsweise ist eine Beschreibung eines Ausführungsbeispiels mit einer Vielzahl von Elementen oder Komponenten nicht dahingehend auszulegen, dass alle diese Elemente oder Komponenten zur Implementierung notwendig sind. Vielmehr können andere Ausführungsbeispiele auch alternative Elemente und Komponenten, weniger Elemente oder Komponenten oder zusätzliche Elemente oder Komponenten enthalten. Elemente oder Komponenten verschiedener Ausführungsbespiele können miteinander kombiniert werden, sofern nichts anderes angegeben ist. Modifikationen und Abwandlungen, welche für eines der Ausführungsbeispiele beschrieben werden, können auch auf andere Ausführungsbeispiele anwendbar sein. Zur Vermeidung von Wiederholungen werden gleiche oder einander entsprechende Elemente in verschiedenen Figuren mit gleichen Bezugszeichen bezeichnet und nicht mehrmals erläutert. Es zeigen:

Fig. 1      eine erste Ausführung eines Endoskops,

Fig. 2      ein distales Ende des Endoskops,

Fig. 3      eine schematische Darstellung einer zweiten Ausführung eines Endoskops,

Fig. 4a, 4b   Darstellungen zum Prinzip einer Vergrößerung eines Linsendurchmessers.

## Ausführliche Beschreibung der Zeichnungen

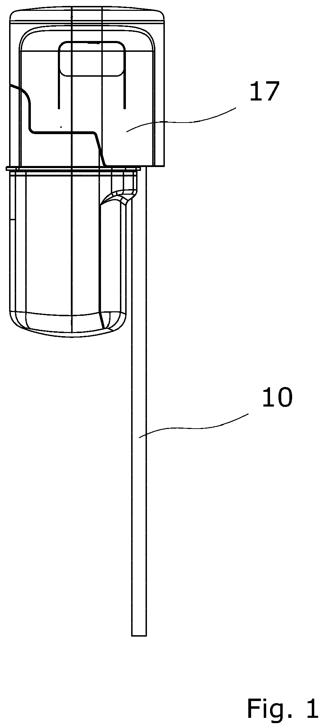

Fig. 1 zeigt ein Endoskop mit einem Endoskopschaft 10 und einem Endoskopgehäuse 17. Innerhalb des Endoskopschafts 10 werden die optischen Bauelemente platziert. Bei einem regulären Stabendoskop trifft das einfallende Licht an einem distalen Ende des Endoskopschafts 10 auf eine Eingangsoptik, wird über verschiedene optische Elemente geformt und anschließend mittels Staboptiken zum distalen Ende des Endoskopschafts 10 geführt, an dem sich das Endoskopgehäuse 17 befindet. Die Bildsensoren sind hier meist in diesem angeordnet. Alternativ können bei sogenannten Chip-on-Tip-Endoskopen die Bildsensoren im Bereich des distalen Endes des Endoskopschafts 10 angeordnet sein. Bei diesem Typ wird das einfallende Licht ebenfalls durch entsprechende optische Bauelemente geformt. Bei Chip-on-Tip-Endoskopen entfällt jedoch die Staboptik, da das Licht direkt auf am distalen Ende des Endoskopschafts 10 angeordnete Sensoren fällt. In der Chirurgie werden meistens Stereoendoskope verwendet, welche zwei getrennte Bildkanäle aufweisen.

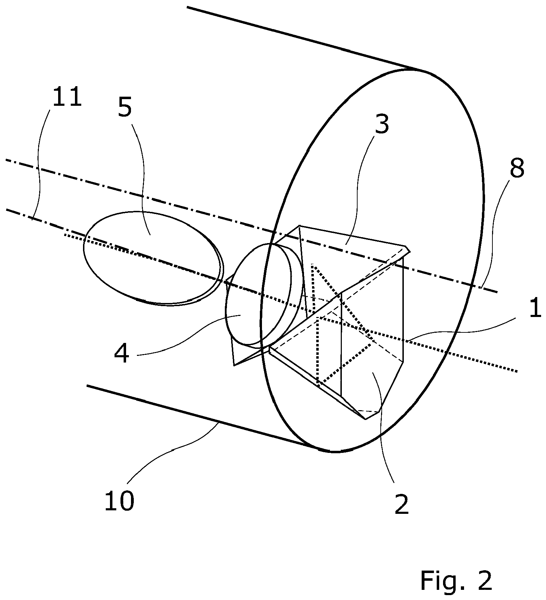

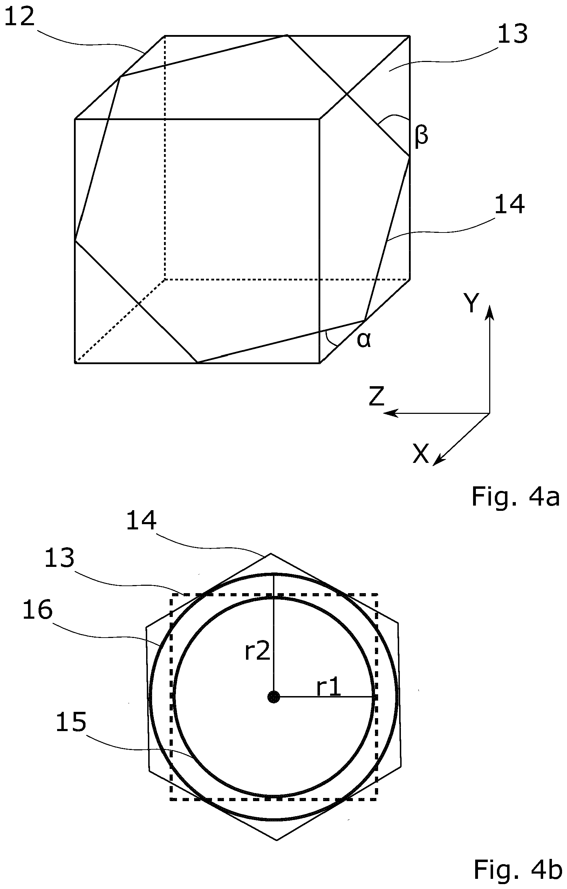

Fig. 2 zeigt das distale Ende eines Endoskops, wobei hier nur einer der beiden Bildkanäle dargestellt ist. Ein Lichtstrahl 1 trifft senkrecht auf ein Prisma, das aus einem ersten Teilprisma 2 und einem zweiten Teilprisma 3 zusammengesetzt ist. Der Lichtstrahl 1 verläuft parallel zu einer Mittelachse 8 des Endoskops, die mittig durch einen Endoskopschaft 10 verläuft, und fällt auf eine Lichteintrittsfläche des ersten Teilprismas 2, die senkrecht zu der Mittelachse 8 und somit auch zu dem Lichtstrahl 1 ausgerichtet ist. Bei dem Prisma handelt es sich in der vorliegenden Ausführungsform um eine Kitgruppe aus dem ersten Teilprisma 2, welches als Bauerfeind-Prisma ausgeführt ist, und dem zweiten Teilprisma 3 in Form eines Schmidt-Prismas. Eine einstückige Ausfertigung ist jedoch ebenso ohne weiteres möglich. Die beiden Teilprismen 2, 3 lenken den Lichtstrahl 1 jeweils mehrfach um und lenken ihn so in zwei Raumebenen um jeweils einen ersten und einen zweiten Prismenwinkel ab. An einer Lichtaustrittsfläche, die sich an der dem ersten Teilprisma 2 abgewandten Seite des zweiten Teilprismas 3 befindet, verlässt der Lichtstrahl 1 das zweite Teilprisma 3 wieder. Die Lichtaustrittsfläche des zweiten Teilprismas 3 schließt mit einer ersten, orthogonal zur Mittelachse 8 ausgerichteten ersten Raumachse einen vorgegebenen ersten Neigungswinkel ein. Ferner schließt die Lichtaustrittsfläche mit einer zweiten, orthogonal zur Mittelachse 8 und zur ersten Raumachse ausgerichteten zweiten Raumachse einen vorgegebenen zweiten Neigungswinkel ein. Diese Anordnung wird später unter Bezugnahme der Fig. 4a und 4b im Detail erläutert. Parallel zu der Lichtaustrittsfläche ist eine Linse 4 mit einer optischen Achse 11 angeordnet, welche senkrecht zur Lichtaustrittsfläche verläuft und die eine größere Querschnittsfläche als die Lichteintrittsfläche aufweist. Der Lichtstrahl 1 trifft im Anschluss auf einen Parabolspiegel 5 als Strahlumlenkungsoptik. Dabei kann es sich insbesondere um einen Off-Axis-Parabolspiegel handeln. Durch diesen wird der Lichtstrahl 1 nun wieder parallel zu der Mittelachse 8 ausgerichtet und zu einem hier nicht gezeigten Sensor oder einem Okular an einem proximalen Ende des Endoskopschafts 10 geführt. Ein Parabolspiegel 5 hat den Vorteil, dass er neben der Umlenkung des Lichtstrahls 1 auch strahlformende Eigenschaften aufweist, um den Lichtstrahl 1 auf die nachfolgenden optischen Elemente, wie lichtleitende Elemente und/oder Sensoren, anzupassen. An Stelle des Parabolspiegels 5 kann auch ein weiteres Prisma eingesetzt werden, um die gewünschte Strahlumlenkung zu erzielen. Zusammen mit der hier nicht gezeigten, optionalen lichtleitenden Optik und dem Sensor bildet der eben beschriebene Aufbau aus Prisma, Linse 4 und Parabolspiegel 5 einen ersten optischen Strahlweg.

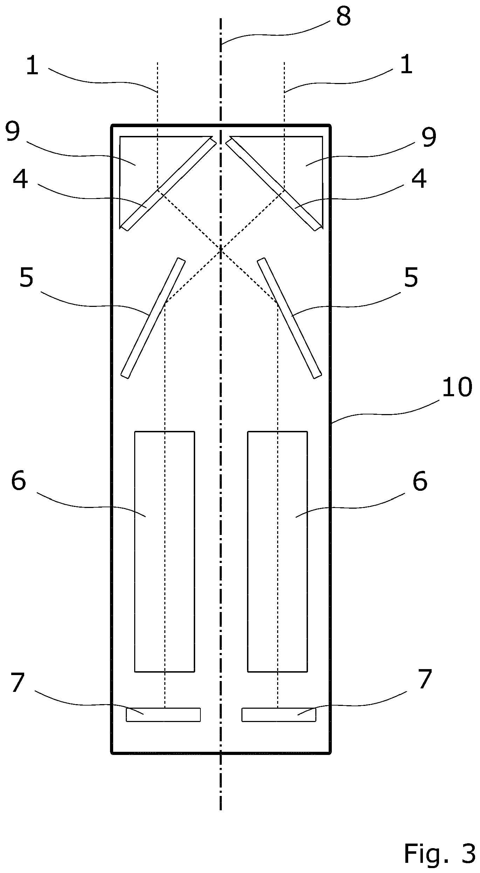

Wird der in Fig. 2 vorgestellte Strahlengang zweifach in einen Endoskopschaft 10 eingebracht, kann ein Stereoendoskop mit zwei Bildkanälen realisiert werden. Ein solches ist in einer schematischen Schnittdarstellung in Fig. 3 gezeigt, bei der das Endoskop einen zweiten optischen Strahlweg, der dem Aufbau des ersten optischen Strahlwegs entspricht, umfasst. Die Anordnung des ersten und des zweiten optischen Strahlwegs erfolgt dabei rotationssymmetrisch zur Mittelachse 8.

Die einfallenden Lichtstrahlen 1 treffen auf die Lichteintrittsflächen der Prismen 9 und werden in Richtung mindestens eines ersten Neigungswinkels abgelenkt. Jedes Prisma 9 ist in diesem Ausführungsbeispiel einstückig gefertigt. Die Lichtstrahlen 1 werden, nachdem sie die Linse 4 durchdrungen haben, in diesem Ausführungsbeispiel in Richtung der Mittelachse 8 geführt, bei der sich die Lichtstrahlen 1 schneiden oder aneinander vorbeigeführt werden. Ein solcher Aufbau erleichtert die Auslegung der verwendeten optischen Elemente trotz des begrenzten Bauraums innerhalb des Endoskopschafts 10. So kann beispielsweise der Krümmungsradius der Linse 4 größer gewählt werden, was Abbildungsfehler reduziert. Wenn sich die Lichtstrahlen 1 kreuzen, kann es grundsätzlich zu Interferenzen kommen, sofern es sich um kohärentes Licht handelt. Da beide Prismen 9 dieselbe Szene aus verschiedenen Perspektiven aufnehmen, um einen Stereoeffekt zu erzielen, spielt dies für das aufgenommene Bild eine untergeordnete Rolle, lediglich das üblicherweise kohärente Beleuchtungslicht kann davon betroffen sein. Da dieses jedoch ungeordnet auf die Lichteintrittsflächen der Prismen 9 fällt, kann eingekoppeltes Streulicht durch Blenden an dem Fokuspunkt der jeweiligen Linse 4 eliminiert werden. Sollten trotzdem noch Interferenzeffekte auftreten, haben sie nur einen geringen Einfluss auf die Bildqualität und können bei Bedarf im Rahmen der elektronischen Bildverarbeitung korrigiert werden. Werden die Lichtstrahlen 1 aneinander vorbeigeführt, können keinerlei Interferenzen mehr auftreten.

Die Lichtstrahlen 1 treffen, nachdem sie die Mittelachse 8 passiert haben, auch in diesem Ausführungsbeispiel auf die Parabolspiegel 5, werden dort parallel zu Mittelachse 8 ausgerichtet und jeweils zu Relayoptiken 6 geleitet, welche das Licht wiederum zum proximalen Ende des Endoskopschafts 10 auf Sensoren 7 lenken.

Wie bereits oben erläutert hat die Linse 4 eine größere Querschnittsfläche als die Lichteintrittsfläche. Der Strahlweg zwischen Linse 4 und dem aufzunehmenden Objekt wird in dem Prisma 9 mehrfach umgelenkt und dadurch verlängert. Durch die um den ersten und den zweiten Neigungswinkel gekippte Anordnung innerhalb des Endoskops kann die Linse 4 größer ausfallen, als es bei einer zur Lichteintrittsfläche parallelen Anordnung der Fall wäre, so dass die numerische Apertur vergrößert wird. Dies soll im Folgenden anhand der Fig. 4a und 4b beschrieben werden.

Zur Vereinfachung ist der mögliche Raum, in dem eine Linse 4 untergebracht werden kann, in Fig. 4a als ein Würfel 12 dargestellt, welcher eine Eintrittsfläche 13 aufweist. Anhand des gezeichneten Koordinatensystems (X,Y,Z) werden die Raumachsen definiert. Das Licht kommt aus Einfallsrichtung Z und trifft senkrecht auf die Eintrittsfläche 13. Eine kreisrunde Linse 4, die innerhalb der Eintrittsfläche 13 liegt, kann maximal einen Durchmesser aufweisen, der der Seitenlänge des Würfels 12 entspricht. Im dreidimensionalen Raum ist es jedoch möglich, eine größere Linse 4 einzubringen, welche vollständig aus der Einfallsrichtung Z betrachtet von der Eintrittsfläche 13 verdeckt wird. Dazu ist die Linse 4 in einer Raumebene anzuordnen, die mit einer ersten Raumachse X einen ersten Neigungswinkel $\alpha$ und einer

zweiten Raumachse Y einen zweiten Neigungswinkel β einschließt. Die erste Raumachse X und die zweite Raumachse Y verlaufen senkrecht zur Einfallsrichtung Z und sind ebenfalls senkrecht zueinander ausgerichtet. Beide Neigungswinkel α, β betragen vorzugsweise 45°, da hier die erreichbare Kreisfläche und damit die mögliche Querschnittsfläche der kreisrunden Linse 4 ein Maximum erreicht.

Bei senkrechtem Einfall des Lichts kann, damit sie vollständig innerhalb der Eintrittsfläche 13 liegt, die Linse 4 einen maximalen Durchmesser aufweisen, der der Seitenlänge des Würfels 12 entspricht. Anders ausgedrückt wird die Querschnittsfläche einer solchen Linse 4 durch einen ersten Kreis 15 beschrieben, der einen ersten Radius r1 aufweist, der der Hälfte der Seitenlänge des Würfels 12 entspricht. Der Flächeninhalt des ersten Kreises 15 berechnet sich zu $\pi*r1^2$.

Die Eintrittsfläche 13 liegt in einer Ebene, welche durch die erste und die zweite Raumrichtung X, Y aufgespannt wird und senkrecht zur Einfallsrichtung Z orientiert ist. Kippt man diese Ebene um den ersten Neigungswinkel α und um den zweiten Neigungswinkel β, hier um jeweils 45°, so ist die in der um die Neigungswinkel α, β gekippten Ebene befindliche und durch den Würfel 12 eingeschlossene Schnittfläche ein Hexagon 14. Aus geometrischen Überlegungen lässt sich herleiten, dass ein vollständig innerhalb des Hexagon 14 liegender zweiter Kreis 16 einen zweiten Radius r2 besitzt, der um den Faktor $\sqrt{3/2}$ gegenüber dem ersten Radius r1 vergrößert ist. Dies entspricht einer Vergrößerung der Querschnittsfläche um 50%. Im Vergleich mit Eintrittsfläche 13 des Würfels 12, welche der Eintrittsfläche des Prismas 9 entspricht, ist die Querschnittsfläche einer solchen Linse 4, die der des zweiten Kreises 16 entspricht, noch immer um den Faktor ($3\pi/8$) vergrößert. Da die numerische Apertur direkt proportional zum Durchmesser der betreffenden Optik ist, kann auf diese Weise auch die numerische Apertur entsprechend vergrößert werden.

**Bezugszeichenliste**

| | |
|---|---|
| 1 | Lichtstrahl |
| 2 | erstes Teilprisma |
| 3 | zweites Teilprisma |
| 4 | Linse |
| 5 | Parabolspiegel |
| 6 | Relayoptik |
| 7 | Sensor |
| 8 | Mittelachse |
| 9 | Prisma |
| 10 | Endoskopschaft |
| 11 | optische Achse |
| 12 | Würfel |
| 13 | Eintrittsfläche |
| 14 | Hexagon |
| 15 | erster Kreis |
| 16 | zweiter Kreis |
| 17 | Endoskopgehäuse |
| X | erste Raumachse |
| Y | zweite Raumachse |
| Z | Einfallsrichtung |
| r1 | erster Radius |
| r2 | zweiter Radius |
| α | erster Neigungswinkel |
| β | zweiter Neigungswinkel |

**Patentansprüche**

1. Endoskop für die laparoskopische Chirurgie,

   - welches einen Endoskopschaft (10) mit einer Mittelachse (8), einem proximalen und einem distalen Ende, und einen ersten optischen Strahlweg mit

      i. einem am distalen Ende des Endoskopschafts (10) angeordneten Prisma (9) mit einer Lichteintrittsfläche und einer Lichtaustrittsfläche,
      ii. einer vom distalen Ende des Endoskopschafts (10) gesehen hinter dem Prisma (9) angeordneten Linse (4) mit einer optischen Achse (11) und
      iii. einer vom distalen Ende des Endoskopschafts (10) hinter der Linse (4) angeordnete Strahlumlenkungsoptik umfasst,

   - wobei die Lichteintrittsfläche des Prismas (9) senkrecht zur Mittelachse (8) ausgerichtet ist, und die Lichtaustrittsfläche des Prismas (9) mit einer ersten, orthogonal zur Mittelachse (8) ausgerichteten ersten Raumachse (X) einen vorgegebenen ersten Neigungswinkel (α) einschließt, **dadurch gekennzeichnet, dass**
   - die optische Achse (11) der Linse (4) senkrecht zur Lichtaustrittsfläche des Prismas (9) angeordnet ist und
   - die Linse (4) eine Querschnittsfläche aufweist, die größer als die Lichteintrittsfläche des Prismas (9) ist.

2. Endoskop nach Anspruch 1, **dadurch gekennzeichnet, dass** der erste Neigungswinkel (α) 45° beträgt.

3. Endoskop nach einem der Ansprüche 1 bis 2, **dadurch gekennzeichnet, dass** das Prisma (9) als Bauernfeind-Prisma oder als Schmidt-Prisma ausgebildet ist.

4. Endoskop nach einem der Ansprüche 1 bis 3, **dadurch gekennzeichnet, dass** die Strahlumlenkungsoptik einen Parabolspiegel (5) mit Off-Axis-Strahlführung und/oder ein weiteres Prisma (9) umfasst, die den Strahlweg parallel zur Mittelachse (8) ausrichten.

5. Endoskop nach einem der Ansprüche 1 bis 4, **dadurch gekennzeichnet, dass** die Lichtaustrittsfläche des Prismas (9) mit einer zweiten, orthogonal zur Mittelachse (8) und zur ersten Raumachse (X) ausgerichteten zweiten Raumachse (Y) einen vorgegebenen zweiten Neigungswinkel ($\beta$) einschließt, der vorzugsweise 45° beträgt.

6. Endoskop nach einem der Ansprüche 1 bis 5, **dadurch gekennzeichnet, dass** das Prisma (9) aus zwei oder mehr Teilprismen (2,3) besteht, welche hintereinander angeordnet, bevorzugt miteinander verkittet sind.

7. Endoskop nach einem der Ansprüche 1 bis 6, **dadurch gekennzeichnet, dass** das Endoskop einen zweiten optischen Strahlweg, der dem Aufbau des ersten optischen Strahlwegs entspricht, umfasst, wobei die Anordnung des ersten und des zweiten optischen Strahlwegs symmetrisch zur Mittelachse (8) erfolgt.

8. Endoskop nach Anspruch 7, **dadurch gekennzeichnet, dass** der erste und der zweite optische Strahlweg sich schneiden oder aneinander vorbeigeführt werden.

Fig. 1

Fig. 2

Fig. 3

Fig. 4a

Fig. 4b

Europäisches
Patentamt

European
Patent Office

Office européen
des brevets

## EUROPÄISCHER RECHERCHENBERICHT

**Nummer der Anmeldung**

**EP 23 15 5157**

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2019/175044 A1 (WINTER & IBE OLYMPUS [DE]) 19. September 2019 (2019-09-19) * Seite 12, Zeile 9 – Zeile 17 * * Seite 13, Zeile 15 – Seite 14, Zeile 29 * * Seite 16, Zeile 12 – Zeile 17 * * Abbildungen 1, 2 * ----- | 1-8 | INV. A61B1/00 A61B1/313 |
| X | DE 10 2015 103214 A1 (STORZ KARL GMBH & CO KG [DE]) 8. September 2016 (2016-09-08) * Absätze [0014], [0060], [0066] – [0068] * * Abbildungen 1, 2 * ----- | 1-8 | |

**RECHERCHIERTE SACHGEBIETE (IPC)**

A61B

Der vorliegende Recherchenbericht wurde für alle Patentansprüche erstellt

| Recherchenort | Abschlußdatum der Recherche | Prüfer |
|---|---|---|
| München | 31. Mai 2023 | Neumeyr, Theresa |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

........................................................................

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**   EP 23 15 5157

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

31-05-2023

| Im Recherchenbericht angeführtes Patentdokument | Datum der Veröffentlichung | Mitglied(er) der Patentfamilie | Datum der Veröffentlichung |
|---|---|---|---|
| WO 2019175044 A1 | 19-09-2019 | DE 102018106220 A1<br>WO 2019175044 A1 | 19-09-2019<br>19-09-2019 |
| DE 102015103214 A1 | 08-09-2016 | KEINE | |

Für nähere Einzelheiten zu diesem Anhang : siehe Amtsblatt des Europäischen Patentamts, Nr.12/82

EPO FORM P0461

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6817975 B1
- DE 102012110905 A1